(19) Europäisches Patentamt
European Patent Office
Office européen des brevets

(11) Veröffentlichungsnummer: **0 211 238**
**B1**

(12) # EUROPÄISCHE PATENTSCHRIFT

(45) Veröffentlichungstag der Patentschrift: **04.04.90**

(51) Int. Cl.$^5$: **C 07 C 215/76,** A 61 K 7/13

(21) Anmeldenummer: **86109037.1**

(22) Anmeldetag: **02.07.86**

(54) **Neue Aminophenole und deren Verwendung in Oxidationshaarfärbemitteln.**

(30) Priorität: **08.07.85 DE 3524329**

(43) Veröffentlichungstag der Anmeldung:
**25.02.87 Patentblatt 87/09**

(45) Bekanntmachung des Hinweises auf die Patenterteilung:
**04.04.90 Patentblatt 90/14**

(84) Benannte Vertragsstaaten:
**AT BE CH DE FR GB IT LI NL SE**

(56) Entgegenhaltungen:
**EP-A-0 023 257**
**EP-A-0 039 030**
**DE-A-1 543 808**
**DE-A-2 628 641**
**CHEMICAL ABSTRACTS, Band 20, Nr. 21, 10. November 1926, Seite 3449, Absatz 2, Columnbus, Ohio, US; H.H. HODGSON et al.: "Nitrosation of phenols. III. Nitrosation of 4-halogeno-0- and m-cresols and oximation of the 4-halogeno-2,5-toluquinones"**

**Die Akte enthält technische Angaben, die nach dem Eingang der Anmeldung eingereicht wurden und die nicht in dieser Patentschrift enthalten sind.**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **Rose, David, Dr.**
**Holbeinweg 7**
**D-4010 Hilden (DE)**
Erfinder: **Lieske, Edgar**
**Hunsrückenstrasse 40**
**D-4000 Düsseldorf (DE)**
Erfinder: **Maak, Norbert, Dr.**
**Im Jagdfeld 41 a**
**D-4040 Neuss (DE)**

EP 0 211 238 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der Erfindung sind neue m-Aminophenole und deren wasserlöslichen Salze. Diese Verbindungen eignen sich in besonders hohem Maße als kupplersubstanzen in Oxidationshaarfärbemitteln.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine Bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate, 4-Aminopyrazolonderivate und Tetraaminopyrimidine eingesetzt. Als sogenannte Kupplersubstanzen werden m-Phenylendiaminderivate, Phenole, Naphthole, Resorcinderivate und Pyrazolone verwendet.

Gute Oxidationsharrfarbstoffvorprodukte müssen in erster Linie folgende Voraussetzungen erfüllen: Sie müssen bei der oxidativen Kupplung die gewünschten Farbnuancen in ausreichender Intensität ausbilden. Diese Farbnuancen müssen gegen die Einwirkung von Wärme, Licht und Chemikalien, z.B. gegen die bei der Kaltwellbehandlung des Haares verwendeten Reduktionsmittel eine ausreichende Stabilität aufweisen. Sie müssen ferner ein gutes Aufziehvermögen auf menschlichem Haar besitzen ohne die Kopfhaut zu stark anzufärben, und sie sollen vor allem in toxikologischer und dermatologischer Hinsicht unbedenklich sein.

Diese Anforderungen werden von den zur Zeit in Oxidationshaarfärbemitteln gebräuchlichen Kupplern, insbesondere von Kupplern, die mit bekannten Entwicklern wie p-Toluyendiamin blaue Nuancen ergeben, nicht zufriedenstellend erfüllt. Besonders problematisch sind die toxikologischen Eigenschaften vieler Kuppler z.B. vom Typ der aromatischen Amine. Andere Kupplersubstanzen führen zu Färbungen mit nicht befriedigenden Echtheitseigenschaften.

Die verwendung von 4-Chlor-3-aminophenol als Kuppler in Oxidationshaarfärbemitteln ist aus DE—A—2509152 bekannt. Die Verwendung von Chlor-methyl-3-aminophenolen, die in 2-Position Chlor oder eine Methylgruppe tragen, als Kuppler in Oxidationshaarfärbemitteln ist aus DE—A—3016008 bekannt. Schließlich ist die Verwendung von 2-Amino-4-hydroxy-5-chlortoluol als Kupplungskomponente für Entwickler vom Typ der aromatischen p-Diamine aus DE—A—1543808 bekannt. Die mit den bekannten m-Aminophenolen als Kuppler herstellbaren Haaranfärbungen befriedigen jedoch nicht in ihren Echtheitseigenschaften.

Es wurde nun gefunden, daß sich 2-Methyl-4-chlor-5-aminphenol und dessen Derivate, die am N-Atom eine Niedrigalkylgruppe oder eine Hydroxyalkylgruppe tragen, als Kuppler zur Erzeugung besonders licht- und reibechter Oxidationshaarfärbemittel eignen. Die genannten m-Aminophenole sind nicht literaturbekannt.

Gegenstand der Erfindung sind daher neue m-Aminophenole der allgemeinen Formel I

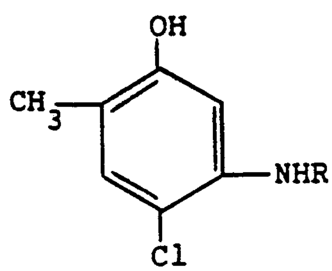

in der R Wasserstoff, eine Alkylgruppe mit 1—4 C-Atomen oder eine Hydroxyalkylgruppe mit 2—4 C-Atomen darstellt sowie deren wasserlösliche Salze.

Die Herstellung der neuen m-Aminophenole erfolgt ausgehend von 4-Chlor-2-methyl-5-nitrophenol, welches aus GB—A—1100219 bekannt ist, durch katalytische Hydrierung zu 4-Chlor-2-methyl-5-aminophenol. Zur Herstellung der erfindungsgemäßen Aminophenole der Formel I, in der R eine Alkyl- oder Hydroxyalkylgruppe darstellt, kann die Gruppe R bach bekanntem Verfahren eingeführt werden. Zum Beispiel lassen sich Alkylgruppen durch Alkylierung mit z.B. Alkyliodid der Formel RI einführen. Die 2-Hydroxyethyl-Gruppe kann eingeführt werden, indem zuerst durch Acylierung mit Chlorameisensäure-2-chlorethylester eine 2-Chlorethoxycarbonyl-Gruppe am Stickstoff eingführt, diese zum 1.3-Oxazolidin-2-on cyclisiert und schließlich zur N-(2-Hydroxyethyl)-Gruppe hydrolisiert.

Bevorzugt, insbesondere wegen seiner leichten Zugänglichkeit, ist das 4-chlor-2-methyl-5-aminophenol. Besonders lichtechte Haaranfärbungen werden jedoch auch mit dem 4-chlor-2-methyl-5-(2-hydroxyethyl)-aminophenol erzielt.

Die erfindungsgemäßen m-Aminophenole können sowohl in freier Form als auch in form ihrer wasserlöslichen Salze hergestellt und eingesetzt werden, z.B. als Hydrochloride, Sulfate, Phosphate, Acetate, Propionate, Lactate oder Citrate.

Ein weiterer Gegenstand der Erfindung ist die Verwendung der neuen m-Aminophenole der allgemeinen Formel I oder deren Salze in Oxidationshaarfärbemitteln als Kupplersubstanzen zusammen mit üblichen Entwicklersubstanzen.

Die erfindungsgemäßen neuen Kupplersubstanzen eignen sich sich für eine Vielzahl veschiedener Entwicklersysteme. Sie erzeugen auf dem Haar tiefblaue, violette, rote oder braune Farbnuancen, je nach dem verwendeten Entwickler. Bevorzugt werden die erfindungsgemäßen m-Aminophenole mit einem Entwickler aus der Gruppe p-Phenylendiamin, p-Toluylendiamin, p-Aminophenol oder 2, 4, 5, 6-Tetraaminopyrimidin eingesetzt.

Ein weiterer Erfindungsgegenstand sind Haarfärbemittel, enthaltend Oxidationsfarbstoffvorprodukte in einem kosmetischen Träger, dadurch gekennzeichnet, daß als Oxidationsfarbstoffvorprodukte Kupplersubstanzen der allgemeinen Formel I in einer Menge von 0,05—10 Millimol pro 100 g des Haarfärbemittels und übliche Entwicklersubstanzen enthalten sind. Als übliche Entwicklersubstanzen sind bevorzugt p-Phenylendiamin, p-Toluylendiamin, p-Aminophenol und/oder 2,4,5,6-Tetraaminopyrimidin in den erfindungsgemäßen Haarfärbemitteln enthalten. Daneben können die erfindungsgemäßen Haarfarbtöne noch andere, bekannte Entwickler, z.B. Derivate der vorgenannten Bevorzugten Entwickler, Diaminopyridinderivate, heterocyclische Hydrazonderivate und 4-Aminopyrazolonderivate, sowie andere bekannte Kuppler enthalten. Als weitere Kuppler eignen sich z.B. m-Phenylendiamine, 2.4-Diaminophenolether, Phenole, Naphthole, Resorcinderivate, z.B. 2-Methylresorcin oder Pyrazolone.

Gegebenenfalls können auch direktziehende Farbstoffe zusätzlich sur weiteren Modifizierung der Farbnuancen eingesetzt werden. Solche direktziehenden Farbstoffe sind z.B. Nitrophenylendiamine, Nitroaminophenole, Anthrachinonfarbstoffe oder Indophenole.

In den erfindungsgemäßen Haarfärbemitteln werden die m-Aminophenole der allgemeinen Formel I und die gegebenenfalls zusätzlich vorhandenen Kupplersubstanzen im allgemeinen in etwa molaren Mengen, bezogen auf die verwendeten Entwicklersubstanzen eingesetzt. Wenn sich auch der molare Einsatz als zweckmäßig erwiesen hat, so ist es jedoch nicht nachteilig, einen gewissen Überschuß einzelner Oxidationsfarbstoffvorprodukte zum Einsatz zu bringen, wobei Entwicklersubstanzen und Kupplersubstanzen in einem Molverhältnis von 1:0,5 bis 1:2 enthalten sein können.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an Haar gewünscht wird. Als Oxidationsmittel kommen insbesondere Wasserstoffperoxid oder dessen Anlagerungsprodukte an Harnstoff, Melamin· oder Natriumborat sowie Gemische aus deratigen Wasserstoffperoxidanlagerungsprodukten mit Kaliumperoxiddisulfat in Betracht.

Zur Herstellung der erfndungsgemäßen Haarfärbemittel werden die Oxidationsfarbstoffvorprodukte in einen geeigneten kosmetischen Träger eingearbeitet. Solche Träger sind zum Beispiel Cremes, Emulsionen, Gele oder auch tensidhaltige, schäumende Lösungen, z.B. Shampoos oder andere Zubereitungen, die für die Anwendung auf dem Haar geeignet sind. Übliche Bestandteile solcher kosmetischer Zubereitungen sind zum Beispiel Netz- und Emulgiermittel wie anionische, nichtionische oder ampholytische Tenside, z.B. Seifen, Fettalkoholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z.B. Methyl- oder Hydroxyethylcellulose, Stärke, Fettalkohole, Paraffinöle, Fettsäuren, ferner Parfümöle und haarpflegende Zusätze, wie z.B. wasserlösliche kationische Polymere, Proteinderivate, Pantothensäure und Cholesterin.

Die Bestandteile der kosmetischen Träger werden zur Herstellung der erfindungsemäßen Haarfärbemittel in für diese Zwecke üblichen Mengen eingesetzt, z.B. werden Emulgiermittel in Konzentrationen von 0,5—30 Gew.-% und Verdickungsmittel in Konzentrationen von 0,1—25 Gew.-% des gesamten Färbemittels eingesetzt. Die Oxidationsfarbstoffvorprodukte werden in Mengen von 0,2—5 Gew.-%, vorzugsweise 1 bis 3 Gew.-% des gesamten Färbemittels in den Träger eingemischt.

Die Anwendung der erfindungsgemäßen Haarfärbemittelkann, unabhängig von der Art der kosmetischen Zubereitung, zum Beispiel als Creme, Gel oder Shampoo im schwach sauren, neutralen oder alkalischen Milieu erfolgen. Bevorzugt ist die Anwendung der Haarfärbemittel in einem pH-Bereich von 8—10. Die Anwendungstemperaturen können in einem Bereich zwischen 15°C und 40°C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z.B. ein Färbeshampoo verwendet wurde.

Die mit den erfindungsgemäßen Haarfärbemitteln erzielbaren Färbungen haben eine hohe Brillanz sowie gute Wärme-, Licht-, Wasch- und Reibechtheitseigenschaften.

Die nachfolgenden Beispiele sollen den Erfindungsgegenstand näher erläutern ohne ihn jedoch hierauf zu beschränken.

## Beispiele

1. 2-Methyl-4-chlor-5-aminophenol-hydrochlorid

5 g 4-Chlor-2-methyl-5-nitrophenol wurden in 200 ml Ethanol gelöst und nach Zugabe von 0,5 g Raney-Nickel katalytisch hydriert. Nach beendeter Wasserstoff-Aufnahme wurde der Katalysator durch Filtration

abgetrennt und das Filtrat mit verdünnter Salzsäure angesäuert. Nach dem Einengen zur Trockene wurden hellbeige Kristalle mit einem Schmelzpunkt von 198°C (unter Zersetzung) erhalten.

2. 2-Methyl-4-chlor-5-ethylaminophenol

Ein Gemisch aus 3 g 4-Chlor-2-methyl-5-aminophenol (0,016 Mol), 2,6 g Äthyliodid (0,017 Mol), 1,35 g Natriumhydrogencarbonat (0,016 Mol) und 30 ml Ethanol wurde 7 Stunden unter Rückflußkühlung zum Sieden erhitzt. Nach dem Abkühlen und Verdünnen mit 50 ml Wasser wurde mit einer Natriumhydrogencarbonat-Lösung neutralisiert und die Lösung dreimal mit je 50 ml Essigsäureethylester extrahiert. Der Extrakt wurde über Natriumsulfat getrocknet und zur Trockene eingeengt. Der Rückstand wurde aus Toluol umkristallisiert. Es wurden beige Kristalle mit einem Schmelzpunkt von 205°C erhalten.

3. 2-Methyl-4-chlor-5-(2-hydroxyethyl)aminophenol

a) [ß-Chlorethyl-(2'chlor-4'methyl-5'hydroxy)phenyl-]-carbamat.

9,7 g 2-Methyl-4-chlor-5-aminophenol-hydrochlorid (0,05 Mol) wurden in 25 ml Dioxan aufgeschlämmt. 5,5 g Calciumcarbonat (0,055 Mol) wurden dazugegeben und die Temperatur auf +90°C erhöht. Unter Rühren wurden dann 7,9 g Chlorameisensäure-2-chlorethylester (0,055 Mol) zugetropft und nach 1 Stunde bei 90°C gerührt. Dann wurde der Ansatz auf +20°C abgekühlt und durch Filtration von den Mineralsalzen abgetrennt. Die filtrierte Lösung wurde mit 100 ml Wasser versetzt und der sich bildende Niederschlag durch Filtration abgetrennt. Es wurden beige Kristalle mit einem Schmelzpunkt von 124°C erhalten.

b) N-[(2'-chlor-4'methyl-5'hydroxy)-phenyl]-1,3-oxazolidin-2-on

10,6 g der Substanz nach 3a (0,04 Mol) wurden in 24 ml 4,3 molare Natronlauge bei 45°C eingetragen. Nach 20 Minuten Rühren wurde das Reaktionsgemisch mit Eiswasser vesetzt und mit verdünnter Salzsäure neutralisiert. Dabei fiel das gewünschte Produkt aus, wurde durch Filtration abgetrennt und bei 70°C getrocknet. Es wurden beige Kristalle mit einem Schmelzpunkt von 198°—200°C erhalten.

c) 2-Methyl-4-chlor-5-(2-hydroxyethyl)-aminophenol

7,3 g der Substanz nach 3b (0,032 Mol) wurden in 20 ml 5 molarer Natronlauge bei 70°C eingetragen. Nach 30 Minuten Reaktionszeit wurde die Lösung auf 0°C abgekühlt und mit konzentrierter Essigsäure, neutralisiert. Das ausfallende Produkt wurde durch Filtration abgetrennt und bei 70°C getrocknet. Es wurden braune Kristalle mit einem Schmelzpunkt von 111°C (unter Zersetzung) erhalten.

Anwendungstechnische Prüfung

Zur Prüfung der neuen Kupplerkomponenten gemäß Beispiel 1—3 wurden diese in Haarfärbe-Cremeemulsionen der folgenden Zusammensetzung eingesetzt:

| | | |
|---|---|---|
| Fettalkohol $C_{12-18}$ | | 10 g |
| Fettalkohol $C_{12-14+}$ 2EO-sulfat, Na-Salz (28 %ig) | | 25 g |
| Wasser | | 60 g |
| Entwicklersubtanz | | 0,0075 Mol |
| Kupplersubstanz | | 0,0075 Mol |
| Inhibitor ($Na_2SO_3$) | | 1,0 g |
| konz. Ammoniak-Lösung | bis | pH = 9,5 |
| Wasser | ad | 100 g |

Die Bestandteile wurden der Reihe nach miteinander vermischt. Nach Zugabe der Entwicklersubstanz und der Kupplersubstanz wurde zunächst mit konzentrierter Ammoniak-Lösung der pH-Wert der Emulsion auf 9,5 eingestellt, dann mit Wasser auf 100 g aufgefüllt.

Die oxidative Kupplung wurde mit 3 %iger Wasserstoffperoxidlösung als Oxidationsmittel durchgeführt. Hierzu wurden 10 g der Färbecreme mit 5 g Wasserstoffperoxidlösung (3 %ig) versetzt und vermischt.

Die Färbecreme wurde auf standardisiertes, zu 90% ergrautes aber nicht besonders vorbehandeltes Menschenhaar aufgetragen und dort 30 Minuten bei 35°C belassen. Nach Beendigung des Färbeprozesses wurde das Haar gespült, mit einem üblichen Haarwaschmittel ausgewaschen und anschließend getrocknet.

Als Eintwicklersubtanzen wurden die folgenden Verbindungen eingesetzt.
E 1: p-Phenylendiamin
E 2: p-Toluylendiamin
E 3: 2-Chlor-p-phenylendiamin
E 4: 2.5-Diaminoanisol
E 5: N-Benzyl-p-phenylendiamin
E 6: N-(2-Hydroxypropyl)-p-phenylendiamin
E 7: N-(p-Aminophenyl)-N',N'-bhis(2-hydroxyethyl)-1,3-diaminopropan
E 8: N,N Dimethyl-p-phenylendiamin
E 9: N,N-Bis-(2-hydroxyethyl)p-phenylendiamin
E 10: 2,5-Diaminobenzylalkohol
E 11: N-Methyl-p-phenylendiamin
E 12: N-(2-Hydroxyethyl)-p-phenylendiamin
E 13: N-(2-Methoxyethyl)-p-phenylendiamin
E 14: N-Butyl-N-sulfobutyl-p-phenylendiamin
E 15: N,N-Diethyl-p-phenylendiamin
E 16: N-Ethyl-N-(2-hydroxyethyl)-p-phenylendiamin
E 17: 2.5-Diaminopyridin
E 18: p-Aminophenol
E 19: 2,4,5,6-Tetraaminopyrimidin
E 20: 2-Dimethylamino-4,5,6-triaminopyrimidin

Als erfindungsgemäße Kupplersubstanzen wurden eingesetzt:
K 1: 2-Methyl-4-chloro-5-aminophenol-hydrochlorid
K 2: 2-Methyl-4-chloro-5-ethylaminophenol
K 3: 2-Methyl-4-chlor-5-(2-hydroxyethyl)-aminophenol

Die Ergebnisse der Haaranfärbungen sind der Tabelle I zu entnehmen.

Für Vergleichsversuche zur Prüfung der Lichtechtheitseigenschaften der Haaranfärbungen wurden folgende, aus DE—A—3016008 bekannten Kupplersubstanzen eingesetzt:
KV 4: 2-Chlor-6-methyl-3-aminophenol
KV 5: 2-Methyl-6-chlor-3-aminophenol

Prüfung der Lichtechtheit

Die Lichtechtheit der gefärbten Haarsträhnen wurde nach DIN 54004 (April 1966) Abschnitt 7.5.2 bestimmt. Die Methode besteht im wesentlichen darin, daß die gefärbten Haarsträhnen nehen Textilproben, die mit 8 blauen, in ihrer Lichtechtheit abgestuften Typfärbungen des Lichtechtheitsmaßstabes mit einer Xenonbogenlampe mit einer Farbtemperatur von 5500 bis 6500°C K belichtet werden. Hierzu werden die Strähnchen und Textilproben nebeneinander auf einem Karton befestigt und die Randzonen der Strähnchen und Textilproben parallel zur Längskante des Probenträgers abgedeckt. Die Belichtung wird unter haufiger Kontrolle durch Abnehmen der Schablone solange durchgeführt, bis Typ 3 des Lichtechtheitsmaßstabes zwischen dem Belichteten und dem unbelichteten Teil einen gerade wahrnehmbaren Unterschied zeigt. Dann stellt man fest, ob die Proben Änderungen zeigen und bewertet diese gegebenenfalls im Vergleich zu den Änderungen der Typen 1, 2 und 3 des Lichtechtheitsmaßstabes. Dann wird weiter belichtet, bis Typ 4 des Lichtechtheitsmaßstabes zwischen dem nunmehr belichteten und dem unbelichteten Teil ebenfalls einen gerade wahrnehmbaren Farbunterschied zeigt. Dann tauscht man die Abdeckplatte durch eine größere aus, die etwa 1/3 der zuvor belichteten Fläche parallel zur Längskante abdeckt. Die Belichtung wird fortgesetzt, bis Typ 6 des Maßstabes einen eben wahrnehmbaren Farbunterschied zeigt. Die Bestimmung der Lichtechtheit erfolgt durch Vergleich der Kontraste auf den Haarsträhnchen mit den Kontrasten auf den Typfärbungen des Lichtechtheitsmaßstabes.

Die Ergebnisse dieser Lichtechtheitsprüfungen sind der Tabelle II zu entnehmen:

Tabelle I

| Beispiel | Entwickler | Kuppler | Nuancen des gefärbten Haares |
|---|---|---|---|
| P 1 | E 1 | K 1 | graugrün |
| P 2 | E 2 | K 1 | dunkelviolett |
| P 3 | E 2 | K 2 | dukelpurpur |
| P 4 | E 2 | K 3 | violett |
| P 5 | E 3 | K 1 | dunkelrubin |
| P 6 | E 4 | K 1 | dunkelviolett |
| P 7 | E 5 | K 1 | dunkelviolett |
| P 8 | E 6 | K 1 | dunkelviolett |
| P 9 | E 7 | K 1 | dunkelpurpur |
| P 10 | E 8 | K 1 | dunkelviolett |
| P 11 | E 9 | K 1 | dunkelviolett |
| P 11 | E 10 | K 1 | dunkelmagenta |
| P 13 | E 11 | K 1 | dunkelviolett |
| P 14 | E 12 | K 1 | dunkelviolett |
| P 15 | E 13 | K 1 | dunkelviolett |
| P 16 | E 14 | K 1 | grauviolett |
| P 17 | E 15 | K 1 | dunkelviolett |
| P 18 | E 16 | K 1 | dunkelviolett |
| P 19 | E 17 | K 1 | graubraun |
| P 20 | E 18 | K 1 | rotbraun |
| P 21 | E 19 | K 1 | mattviolett |
| P 22 | E 20 | K 1 | dunkelblau |

Tabelle II

| Beispiel | Entwickler | Kuppler | Nuance | Lichtechtheitsnote |
|---|---|---|---|---|
| V 1 | E 2 | K 1 | dunkelviolett | 6 |
| V 2 | E 2 | KV 4 | blauviolett | 5 |
| V 3 | E 2 | KV 5 | dunkelviolet | 4 |

**Patentansprüche**

1. m-Aminophenole der allgemeinen Formel I

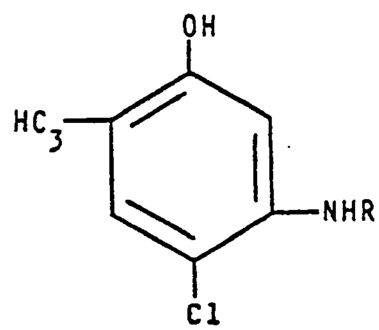

in der R Wasserstoff, eine Alkylgruppe mit 1—4 C-Atomen oder eine Hydroxyalgruppe mit 2—4 C-Atomen darstellt sowie deren wasserlösliche Salze.

2. m-Aminophenole nach Anspruch 1, dadurch gekennzeichnet, daß R Wasserstoff ist.

3. Verfahren zur Herstellung von m-Aminophenolen der allgemeinen Formel I nach Anspruch 1, dadurch gekennzeichnet, daß man 4-Chlor-2-methyl-5-nitrophenol katalytisch zum 4-Chlor-2-methyl-5-aminophenol hydriert und gegebenenfalls an der Aminogruppe die Gruppe R nach bekannten Verfahren einführt.

4. Verwendung der m-Aminophenole nach Anspruch 1 oder 2 oder deren Salzen in Oxidationshaarfärbemitteln als Kupplersubstanzen zusammen mit üblichen Entwicklersubstanzen.

5. Verwendung nach Anspruch 4, dadurch gekennzeichnet, daß als Entwicklersubstanzen p-Phenylendiamin, p-Toluylendiamin, p-Aminophenol oder 2,4,5,6-Tetraaminopyrimidin eingesetzt werden.

6. Haarfärbemittel, enthaltend Oxidationsfarbstoffvorprodukte in einem kosmetischen Träger, dadurch gekennzeichnet, daß als Oxidationsfarbstoffvorprodukte Kupplersubstanzen der allgemeinen Formel I nach Anspruch 1 oder 2 in einer Menge von 0,05—10 Millimol pro 100 g des Haarfärbemittels und übliche Entwicklersubstanzen enthalten sind.

7. Haarfärbemittel nach Anspruch 6, dadurch gekennzeichnet, daß zusätzlich weitere, bekannte Kupplersubstanzen enthalten sind, daß Entwicklersubstanzen und Kupplersubstanzen im Molverhältnis 1:0,5 bis 1:2 enthalten sind und daß der Gehalt an Oxidationsfarbstoffvorprodukten 0,2—5,0 Gew.-% des Haarfärbemittels beträgt.

**Revendications**

1. m-aminophénols de formule générale I

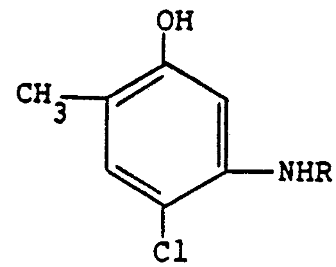

dans laquelle R représente un atome d'hydrogène, un groupe alkyle ayant de 1 à 4 atomes de carbone ou un groupe hydroxyalkyle ayant de 2 à 4 atomes de carbone, et sels solubles dans l'eau de ceux-ci.

2. m-aminophénols selon la revendication 1, caractérisés en ce que R est un atome d'hydrogène.

3. Procédé pour la préparation de m-aminophénols de formule générale I selon la revendication 1, caractérisé en ce que l'on soumet à une hydrogénation catalytique du 4-chloro-2-méthyl-5-nitrophénol pour aboutir au 4-chloro-2-méthyl-5-aminophénol et on introduit éventuellement, selon des procédés connus, le groupe R sur le groupe amino.

4. Utilisation des m-aminophénols selon la revendication 1 et 2, ou de leurs sels, dans des produits de teinture pour cheveux par oxydation, en tant que substances de copulation, conjointement avec des substances usuelles de développement.

5. Utilisation selon la revendication 4, caractérisée en ce que l'on utilise en tant que substances de développement la p-phénylènediamine, la p-toluylènediamine, le p-aminophénol ou la 2,4,5,6-tétraaminopyrimidine.

6. Produit de teinture pour cheveux contenant des précurseurs de colorants d'oxydation dans un véhicule cosmétique, caractérisé en ce qu'il contient, en tant que précurseurs de colorants d'oxydation, des substances de copulation de formule générale I selon les revendications 1 ou 2, en une quantité de 0,05—10 mmoles pour 100 g du produit de teinture pour cheveux, et des substances usuelles de développment.

7. Produit de teinture pour cheveux selon la revendication 6, caractérisé en ce qu'il contient en outre d'autres substances de copulation connues, en ce que les substances de développement et les substances de copulation sont contenues en un rapport molaire allant de 1:0,5 à 1:2, et en ce que la teneur en précurseurs de colorants d'oxydation est de 0,2 à 5,0% en poids du produit de teinture pour cheveux.

**Claims**

1. m-aminophenols corresponding to the following general formula

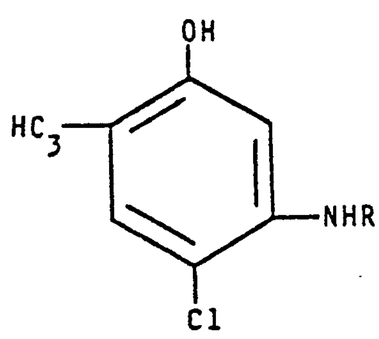

in which R is hydrogen, a $C_1$—$C_4$ alkyl group or a $C_2$—$C_4$ hydroxyalkyl group, and water-soluble salts thereof.

2. m-aminophenols as claimed in Claim 1, characterized in that R is hydrogen.

3. A process for preparing the m-aminophenols corresponding to general formula I in Claim 1, characterized in that 4-chloro-2-methyl-5-nitrophenol is catalytically hydrogenated to 4-chloro-2-methyl-5-aminophenol and the group R is optionally introduced in known manner at the amino group.

4. The use of the m-aminophenols claimed in Claim 1 or 2 or salts thereof as couplers together with standard developers in oxidation hair dyes.

5. The use claimed in Claim 4, characterized in that p-phenylene diamine, p-tolylene diamine, p-aminophenol or 2,4,5,6-tetraaminopyrimidine are used as developers.

6. Hair dyes containing oxidation dye precursors in a cosmetic carrier, characterized in that they contain as oxidation dye precursors couplers of general formula I as claimed in Claim 1 or 2 in a quantity of from 0.05 to 10 millimoles to 100 g of the hair dye and standard developers.

7. Hair dyes as claimed in Claim 6, characterized in that other known couplers are additionally present, in that developers and couplers are present in a molar ratio of from 1:0.5 to 1:2 and in that the content of oxidation dye precursors amounts to between 0.2 and 5.0% by weight, based on the hair dye.